Europäisches Patentamt

European Patent Office

Office européen des brevets

(11) Publication number: **0 192 319 B1**

## EUROPEAN PATENT SPECIFICATION

(45) Date of publication of patent specification: **15.07.92** (51) Int. Cl.5: **A01N 63/00**

(21) Application number: **86300128.5**

(22) Date of filing: **09.01.86**

(54) **Cellular encapsulation of biological pesticides.**

(30) Priority: **22.01.85 US 693080**
**28.06.85 US 750369**
**30.08.85 US 771313**

(43) Date of publication of application:
**27.08.86 Bulletin 86/35**

(45) Publication of the grant of the patent:
**15.07.92 Bulletin 92/29**

(84) Designated Contracting States:
**BE CH DE FR GB IT LI NL SE**

(56) References cited:
**EP-A- 0 063 949**
**EP-A- 0 093 062**
**EP-A- 0 185 005**
**EP-A- 0 200 344**

**JOURNAL OF INVERTEBRATE PATHOLOGY, vol. 25, 1975, pages 355-361, Academic Press, Inc.; J. NISHIITSUTSUJI-UWO et al.: "Sporeless mutants of Bacillus thuringiensis"**

(73) Proprietor: **Mycogen Corporation**
**5451 Oberlin Drive**
**San Diego, CA 92121(US)**

(72) Inventor: **Barnes, Andrew C.**
**13306 Landfair Road**
**San Diego, CA 92121(US)**
Inventor: **Cummings, Susan G.**
**12214 Beach Avenue**
**Beach Haven New Jersey 08008(US)**

(74) Representative: **Perry, Robert Edward et al**
**GILL JENNINGS & EVERY 53-64 Chancery Lane**
**London WC2A 1HN(GB)**

EP 0 192 319 B1

JOURNAL OF INVERTEBRATE PATHOLOGY, vol. 22, 1973, pages 273-277, Academic Press, Inc.; H.T. DULMAGE: "HD-187, a new isolate of Bacillus thuringiensis that produces high yields of delta-endotoxin"

CHEMICAL ABSTRACTS, vol. 97, 8th November 1982, page 198, abstract no. 157405m, Columbus, Ohio, US; H.R. WHITELEY et al.: "Cloning the crystal protein gene of B. thuringiesis in E. coli", & MOL. CLONING GENE REGUL. BACILLI 1982, 131-44

ENGINEERED ORGANISMS IN THE ENVIRON-MENT, 1985, pages 40-46, American Society for Microbiology, Washington, US; L. WATRUD et al.: "Cloning of the Bacillus thuringiensis subsp. Kurstaki delta-endotoxin gene into Pseudomonas fluorescens: Molecular biology and ecology of an engineered microbial pesticide"

GENETIC ENGINEERING NEWS, vol. 5, no. 2, February 1985, pages 1,8, Mary Ann Liebert, Inc, New York, US; J. STERLING: "Monsanto to perform first bio-pesticide field test"

**Description**

The extraordinary increase in agricultural productivity has been a result of many factors, including significantly better understanding of the methods involved with agriculture, improved equipment, availability of fertilizers, and improved pesticides. The latter factor has not been without detrimental aspects, however, due to the negative effect on the environment. There is, therefore, a substantial interest in developing effective and environmentally acceptable pesticides.

Among ecologically acceptable pesticides are the protein toxins produced by various microorganisms, such as Bacillus thuringiensis. However, the use of B. thuringiensis lysate or spores as a pesticide has significant drawbacks. The lifetime of the pesticide is relatively short in the environment, requiring multiple applications to give adequate protection. Consequently, these pesticides are not economical in comparison to more traditional chemical products having long residual activities. Improvements in field longevity would greatly aid in expanding the application of biological, or protein toxin-based pesticides.

As indicated above, there are many requirements for pesticides associated with their particular application. For example, in many cases it is desirable to have pesticides which have long residual activity in the field while not accumulating in the environment. In addition, because of economic considerations, it is preferable to have pesticides which have a reasonably broad spectrum of biocidal activity. Also, the pesticide should degrade to degradation products which are environmentally-acceptable. Other considerations include ease of formulation, pesticidal activity, stability to environmental effects such as light, water and organisms, and the effect on beneficial or innocuous organisms in the environment.

West, Soil Biol. Biochem. (1984) 16:357-360, reports the results of a study on the persistence of B.t. toxin in soil. See also West et al, J. of Invertebrate Pathology (1984) 43:150-155. US-A-4265880 describes embedding live insecticidal pathogens in a coacervate microbead. Japanese Patent No. 51-5047 describes physical-chemical methods for killing B. thuringiensis spores, while retaining toxicity.

Nishiitsutsuji-Uwo et al, J. Invertebrate Pathology (1975) 25:355-361, disclose heat-killed cells of sporeless mutants of Bacillus thuringiensis, and that the heat treatment kills the cells and causes a slight but insignificant reduction of toxicity.

EP-A-0063949 states that B. thuriengiensis produces a crystal protein during sporulation, and genetically-engineered bacterial host strains that express the crystal protein without growth phase limitations.

According to the present invention, substantially intact, non-reproducing microbial cells contain a pesticide which is a polypeptide, is intracellular and is produced naturally or as a result of a heterologous gene in the cell; the cells are stabilised, by treatment with suitable reagents, under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants.

In accordance with the subject invention, improved pesticides are provided, having among their other advantages an extended residual life, by modifying naturally-occurring pesticide-producing microorganisms, or pesticide-producing microorganisms hosting a heterologous gene capable of expression in the host, where expression of the gene results, directly or indirectly, in the production of a pesticide. The subject invention involves treating the organisms with reagents that prolong the activity of the toxin produced in the cell when the cell is applied to the environment of target pest(s). The resulting product retains the toxicity of the toxin.

A wide variety of pesticides can be produced which will be characterized by being capable of being produced intracellularly, particularly in a unicellular microorganism host, such as prokaryotes, e.g., bacteria; or eukaryotes, e.g., fungi, exemplified by yeast and filamentous fungi, such as Neurospora and Aspergillus; or protists, such as amoebas, protozoa, algae, and the like.

The pesticide can be any toxin produced by a microbe. For example, it can be a polypeptide which has toxic activity toward a eukaryotic multicellular pest, such as insects, e.g., coleoptera, lepidoptera, diptera, hemiptera, dermaptera, and orthoptera; or arachnids; gastropods; or worms, such as nematodes and platyhelminths. Various susceptible insects include beetles, moths, flies, grasshoppers, lice, and earwigs.

The pesticide which is produced in the host cell may be a polypeptide produced in active form or a precursor or proform which requires further processing for toxin activity, e.g., by the pest, as with the crystal toxin of B. thuringiensis var. kurstaki. Thus, the gene may encode an enzyme which modifies a metabolite to produce a pesticidal composition.

Among naturally-occurring toxins are the polypeptide crystal toxins of B. thuringiensis var. kurstaki, active against lepidoptera; B.t. var. israelensis, active against mosquitoes; B.t. M-7, active against coleop-

3

tera; B. thuringiensis var. aizawai, active against spodoptera; and B. sphaericus, active against mosquito larvae. Other toxins include those of entomopathogenic fungi, such as beauverin of Beauveria bassiana and destruxins of Metarhizium spp.; or the broad spectrum insecticidal compounds, such as the avermectins of Streptomyces avermitilus. Cultures exemplifying the above are as follows:

Bacillus thuringiensis var. kurstaki HD-1--NRRL B-3792; disclosed in U.S. Patent 4,448,885
Bacillus thuringiensis var. israelensis--ATCC 35646
Bacillus thuringiensis M-7--NRRL B-15939
Bacillus thuringiensis var. tenebrionis--DSM 2803

The following B. thuringiensis cultures are available from the United States Department of Agriculture (USDA) at Brownsville, Texas. Requests should be made to Joe Garcia, USDA, ARS, Cotton Insects Research Unit, P.O. Box 1033, Brownsville, Texas 78520 USA.

B. thuriniensis HD2
B. thuringiensis var. finitimus HD3
B. thuringiensis var. alesti HD4
B. thuringiensis var. kurstaki HD73
B. thuringiensis var. sotto HD770
B. thuringiensis var. dendrolimus HD7
B. thuringiensis var. kenyae HD5
B. thuringiensis var. galleriae HD29
B. thuringiensis var. canadensis HD224
B. thuringiensis var. entomocidus HD9
B. thuringiensis var. subtoxicus HD109
B. thuringiensis var. aizawai HD11
B. thuringiensis var. morrisoni HD12
B. thuringiensis var. ostriniae HD501
B. thuringiensis var. tolworthi HD537
B. thuringiensis var. darmstadiensis HD146
B. thuringiensis var. toumanoffi HD201
B. thuringiensis var. kyushuensis HD541
B. thuringiensis var. thomosoni HD542
B. thuringiensis var. pakistani HD395
B. thuringiensis var. israelensis HD567
B. thuringiensis var. indiana HD521
B. thuringiensis var. dakota
B. thuringiensis var. tohokuensis HD866
B. thuringiensis var. kumanotoensis HD867
B. thuringiensis var. tochigiensis HD868
B. thuringiensis var. colmeri HD847
B. thuringiensis var. wuhanensis HD525
Bacillus cereus--ATCC 21281
Bacillus moritai--ATCC 21282
Bacillus popilliae--ATCC 14706
Bacillus lentimorbus--ATCC 14707
Bacillus sphaericus--ATCC 33203
Beauveria bassiana--ATCC 9835
Metarhizium anisopliae--ATCC 24398
Metarhizium flavoviride--ATCC 32969
Streptomyces avermitilus--ATCC 31267

The toxin need not be the same as a naturally-occurring toxin. Polypeptide toxins may be fragments of a naturally-occurring toxin; expression products of deletion, transversion or transition mutations, where two or fewer number percent of the amino-acids may be changed; or a repetitive sequence capable of processing by the intended pest host. In addition, fusion products may be prepared where one, five or more amino-acids are provided at the N-terminus to provide, for example, reduced proteolytic degradation of the toxin(s). In some instances, a plurality of the same or different toxins may be encoded and expressed, where processing sites may be introduced between each toxin moiety in the polytoxin.

Illustrative host cells may include either prokaryotes or eukaryotes, normally being limited to those cells which do not produce substances toxic to higher organisms, such as mammals. However, organisms which produce substances toxic to higher organisms could be used, where the toxin is unstable or the level of

4

application sufficiently low as to avoid any possibility of toxicity to a mammalian host. As hosts, of particular interest will be the prokaryotes and lower eukaryotes, such as fungi. Illustrative prokaryotes, both Gram-negative and -positive, include Enterobacteriaceae, such as Escherichia, Erwinia, Shigella, Salmonella, and Proteus; Bacillaceae; Rhizobiaceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the heterologous gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such Pseudomonas sp., Erwinia sp. and Flavobacterium sp.; or such other organisms as Escherichia, Lactobacillus sp., Bacillus sp., and the like. Specific organisms include Pseudomonas aeruginosa, Pseudomonas fluorescens, Saccharomyces cerevisiae, Bacillus thuringiensis, Escherichia coli, Bacillus subtilis, and the like.

The cell will usually be intact and be substantially in the proliferative form when killed, rather than in a spore form, although in some instances spores may be employed.

The cells may be inhibited from proliferation in a variety of ways, so long as the technique does not deleteriously affect the properties of the pesticide, nor diminish the cellular capability in protecting the pesticide. The techniques may involve physical treatment, chemical treatment, changing the physical character of the cell or leaving the physical character of the cell substantially intact, or the like.

Various techniques for inactivating the host cells include heat, usually 50°C to 70°C; freezing; UV irradiation; lyophilization; toxins, e.g., antibiotics; phenols; anilides, e.g., carbanilide and salicylanilide; hydroxyurea; quaternaries; alcohols; antibacterial dyes; EDTA and amidines; non-specific organic and inorganic chemicals, such as halogenating agents, e.g., chlorinating, brominating or iodinating agents; aldehydes, e.g., glutaraldehyde or formaldehyde; toxic gases, such as ozone and ethylene oxide; peroxide; psoralens; desiccating agents; or the like,which may be used individually or in combination. The choice of agent will depend upon the particular pesticide, the nature of the host cell, the nature of the modification of the cellular structure, such as fixing and preserving the cell wall with crosslinking agents, or the like.

The cells generally will have enhanced structural stability which will enhance resistance to environmental degradation in the field. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bioavailability or bioactivity of the toxin.

The heterologous gene(s) may be introduced into the host in any convenient manner, either providing for extrachromosomal maintenance or integration into the host genome. (By heterologous is intended that the gene is not present in the host into which it is introduced, nor would the gene normally be found in such host. That is, even if the host organism and the source of the heterologous gene exchange information, the heterologous gene would normally not be found in the wild-type host cells in nature. Usually, the term heterologous will involve species of different genera as host and gene source.)

Various constructs may be used, which include replication systems from plasmids, viruses, or centromeres in combination with an autonomous replicating segment (ars) for stable maintenance. Where only integration is desired, constructs can be used which may provide for replication, and are either transposons or have transposon-like insertion activity or provide for homology with the genome of the host. Frequently, DNA sequences are employed having the heterologous gene between sequences which are homologous with sequences in the genome of the host, either chromosomal or plasmid. Desirably, the heterologous gene(s) will be present in multiple copies. See for example, U.S. Patent No. 4,399,216. Thus, conjugation, transduction, transfection and transformation may be employed for introduction of the heterologous gene.

A large number of vectors are presently available which depend upon eukaryotic and prokaryotic replication systems, such as ColEl, P-1 incompatibility plasmids, e.g., pRK290, yeast 2m $\mu$ plasmid,

lambda, and the like.

Where an extrachromosomal element is employed, the DNA construct will desirably include a marker which allows for a selection of those host cells containing the construct. The marker is commonly one which provides for biocide resistance, e.g., antibiotic resistance or heavy metal resistance, complementation providing prototrophy to an auxotrophic host, or the like. The replication systems can provide special properties, such as runaway replication, can involve cos cells, or other special feature.

Where the heterologous gene(s) has transcriptional and translational initiation and termination regulatory signals recognized by the host cell, it will frequently be satisfactory to employ those regulatory features in conjunction with the heterologous gene. However, in those situations where the heterologous gene is modified, as for example, removing a leader sequence or providing a sequence which codes for the mature form of the pesticide, where the entire gene encodes for a precursor, it will frequently be necessary to manipulate the DNA sequence, so that a transcriptional initiation regulatory sequence may be provided which is different from the natural one.

A wide variety of transcriptional initiation sequences exist for a wide variety of hosts. The sequence can provide for constitutive expression of the pesticide or regulated expression, where the regulation may be inducible by a chemical, e.g., a metabolite, by temperature, or by a regulatable repressor. See for example, U.S. Patent No. 4,374,927. The particular choice of the promoter will depend on a number of factors, the strength of the promoter, the interference of the promoter with the viability of the cells, the effect of regulatory mechanisms endogenous to the cell on the promoter, and the like. A large number of promoters are available from a variety of sources, including commercial sources.

The cellular host containing the heterologous pesticidal gene may be grown in any convenient nutrient medium, where the DNA construct provides a selective advantage, providing for a selective medium so that substantially all or all of the cells retain the heterologous gene. These cells may then be harvested in accordance with conventional ways and modified in the various manners described above. Alternatively, the cells can be fixed prior to harvesting.

The method of treating the host organism containing the toxin can fulfill a number of functions. First, it may enhance structural integrity. Second, it may provide for enhanced proteolytic stability of the toxin, by modifying the toxin so as to reduce its susceptibility to proteolytic degradation and/or by reducing the proteolytic activity of proteases naturally present in the cell. The cells are preferably modified at an intact stage and when there has been a substantial build-up of the toxin protein. These modifications can be achieved in a variety of ways, such as by using chemical reagents having a broad spectrum of chemical reactivity. The intact cells can be combined with a liquid reagent medium containing the chemical reagents, with or without agitation at temperatures in the range of about -10 to 60°C. The reaction time may be determined empirically and will vary widely with the reagents and reaction conditions. Cell concentrations will vary from about $10^2$ to $10^{10}$ per ml.

Of particular interest as chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that prolong the activity of the toxin produced in the cell when the cell is applied to the environment of the target pest(s).

For halogenation with iodine, temperatures will generally range from about 0 to 50°C, but the reaction can be conveniently carried out at room temperature. Conveniently, the iodination may be performed using triiodide or iodine at 0.5 to 5% in an acidic aqueous medium, particularly an aqueous carboxylic acid solution that may vary from about 0.5-5M. Conveniently, acetic acid may be used, although other carboxylic acids, generally of from 1 to 4 carbon atoms, may also be employed. The time for the reaction will generally range from less than a minute to 24 hrs, usually from 1 to 6 hrs. Any residual iodine may be removed by reaction with a reducing agent, such as dithionite, sodium thiosulfate, or other reducing agent compatible with ultimate usage in the field. In addition, the modified cells may be subjected to further treatment, such as washing to remove all of the reaction medium, isolation in dry form, and formulation with typical stickers, spreaders, and adjuvants generally utilized in agricultural applications, as is well known to those skilled in the art.

Of particular interest are reagents capable of crosslinking the cell wall. A number of reagents are known in the art for this purpose. The treatment should result in enhanced stability of the pesticide. That is, there should be enhanced persistence or residual activity of the pesticide under field conditions. Thus, under conditions where the pesticidal activity of untreated cells diminishes, the activity of treated cells remains for periods of from 1 to 3 times longer.

The cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants or polymers.

Cells of the invention may be formulated as a concentrate or as a pesticidal formulation to be used directly. A formulation may contain from 1 to 100% by weight of the pesticide. A dry formulation may contain from 1 to 95% by weight of the pesticide. A liquid formulation will generally contain from 1 to 60% by weight solids. The formulation generally has from $10^2$ to $10^4$ cells/mg.

The formulations can be applied to the pest environment, such as plants, soil or water, e.g. by spraying, dusting or sprinkling. 0.05 to 1 kg (liquid or dry), or more, of formulations such as those described above may be applied per hectare.

The following Examples illustrate the invention and its utility.

In Example 1, intact spore-containing cells are treated with lugol's iodine and then killed. Here and in general, since the cells are naturally-occurring, killing is not necessary to obtain the benefits of the invention, Treatment of the cells, as described herein, can be conducted by a person skilled in the art, to optimise the length of toxin (pesticidal) activity in the environment of the target pest(s).

Example 1

Intact cells of Bacillus thuringiensis (HD-1) were harvested just prior to autolysis of the sporulating cells, by centrifugation; the cell pellet was suspended in deionised water to give a concentration of 6.0 x $10^9$ cells/ml. An aliquot of the cell suspension was diluted to 1.5 x $10^8$ cells/ml and exposed to 1% lugol's iodine for 4 hr at room temperature (1% lugol's solution contains 1.0 g potassium iodide, 0.5 g iodine and 1.0 ml glacial acetic acid per litre). The treated cells were washed and resuspended in sterile deionised water to give a cell concentration of 6.0 x $10^9$. No viable treated cells were detected by plate counts on nutrient agar.

In a bioassay, various dilutions of the treated, killed cells and also of untreated live HD-1 cells were mixed with a constant volume of larval diet cup. A single 5-day old T. ni larva was then added to each cup. Eighteen larvae were tested per dilution. The percentages of larvae killed after six days are shown in Table 1.

Table 1

| Cell Dilution | $10^9$ | $10^8$ | $10^7$ | $10^6$ | $10^5$ |
|---|---|---|---|---|---|
| Untreated | 100 | 100 | 68.8 | 0 | 0 |
| Treated | 94.4 | 61.0 | 0 | 0 | 0 |

Example 2--Stability Testing

Intact, spore-containing cells of B.t. HD-1 were treated with 1% lugol's solution for 4 hr at room temperature, washed in deionized water, and stored in the refrigerator for 52 days. After this period the cells remained whole, and there was no evidence of lysis (release of spores and crystal).

Intact, spore-containing cells of B.t. HD-1 were harvested by centrifigation and the resulting pellet suspended in sterile deionized water ($10^{10}$ cells/ml), heated to 70°C for 30 min, and stored in the refrigerator for 9 days. After this period, virtually all of the cells have lysed, releasing spores and crystals.

Example 3

Soil Experiment Procedure

B.t. HD-1 preparation:

An intact spore-containing culture of B.t. HD-1 was harvested by centrifugation and the cell pellet resuspended in 400 ml of 1% lugol's iodine (4 x $10^8$ cells/ml). The iodine-cell suspension was stirred for 3 hr at room temperature, washed 3 times and resuspended in 400 ml sterile 0.1 M sodium phosphate buffer, pH 6.9. No viable B.t. HD-1 cells were detected on nutrient agar ($10^1$) after treatment with iodine, and microscopic examination revealed that all cells were intact (unlysed).

Dipel (0.1 g, Abbott Laboratories, 16,000 International Units of Potency/mg. List #5188), which contains B.t. HD-1 cells, was measured into 400 ml of sterile 0.1 M sodium phosphate buffer.

1) Non-sterile soil preparation: 40 g of soil was placed in a sterile 500 ml flask and 200 ml of experimental solution added.

2) Sterile soil preparation: 40g of soil was placed in a sterile 500ml flask and autoclaved for 1 hr prior to adding 200ml of experimental solution. Flasks containing soil suspensions were incubated on a gyratory shaker (200RPM) at room temperature. Samples (30-40ml) of each soil suspension were filtered through 4x cheesecloth and sprayed onto the leaves of lettuce plants for subsequent measurement of toxicity against larvae of T. ni.

Leaves of Romaine lettuce seedlings are sprayed with freshly prepared standard concentrations of Dipel (1x 0.19g/100ml, 1/10x, 1/20x, and 1/100x), and experimental solutions 24 hr before use.

Leaves treated with standards or experimental solutions are removed from the plant, weighed individually, and placed in individual petri dishes. Ten starving, 7 day old T. ni larvae are applied to each leaf and allowed to feed on the leaf for approximately 24 hrs at 25°C. At the end of the feeding period the leaves are re-weighed and the average weight loss determined for each treatment.

Under the conditions described above, leaf weight loss is a log linear function of toxin concentration over concentrations of toxin equivalent to 0.19 mg/ml-0.019 mg/ml Dipel (16,000 international units of potency per mg). Thus, the freshly-prepared Dipel concentrations sprayed onto lettuce plants and run with each assay serve as standards by which the concentration of B.t. HD-1 toxin on leaves sprayed with experimental solutions can be equated. The data in Table 2 show the percentage of the original toxicity (day 1) remaining at various times after incubation in soil and subsequently assayed with the leaf inhibition assay. Leaves other than lettuce plant leaves can be used in assaying for B.t. toxin efficacy.

Table 2

Persistence of Toxicity in Soil

% of Original Toxicity Remaining

| Days After Start of Soil Incubation | Dipel (Spore Crystal Preparation) | | Pre-Lysed Lugol's Iodine Treated B.t. HD-1 (Whole Cell Preparation) | |
|---|---|---|---|---|
| | Sterile | Nonsterile | Sterile | Nonsterile |
| 1 | 100 | 100 | 100 | 100 |
| 5 | 81 | 0.5 | 133 | 100 |
| 8 | 74 | 5.3 | 80 | 76 |
| 15 | - | - | 87 | 117 |

Inactivation of the protein-crystal of B.t. HD-1 in soil has been shown to be due to the activity of soil microorganisms (West, 1984). The results of Table 2 demonstrate that when Dipel and a lugol's iodine-treated pre-lysed B.t. HD-1 preparation were incubated under similar conditions, the toxicity of the Dipel (spore-crystal) preparation degenerated rapidly, whale the toxicity of the lugol's iodine-treated cells remained essentially unchanged

It is evident from the above results that chemical treatment of whole microbial cells can be performed in such a way as to retain polypeptide toxin activity, while rendering the cell stable to storage conditions. This provides increased residual activity of toxin activity under field conditions.

Example 4--Construction of a Heterologous Gene and Transformation into A Suitable Host.

A construction began with a clone of Pseudomonas aeruginosa, available from Northern Regional Research Laboratories (NRRL B-12127), containing a broad host range shuttle plasmid pRO1614 (J. Bact. [1982] 150:60; U.S. Patent No. 4,374,200). The plasmid has unique HindIII, BamHI, and SalI and PvuII restriction sites, a PstI insertion, which includes the carbenicillin resistance gene and a P. aeruginosa replication system, where the HindIII, BamHi and SalI restriction sites are in a tetracycline resistance gene. The remainder of the plasmid is derived from pBR322. A second plasmid, pSM1-17, has been deposited as a clone of E. coli (NRRL B-15976). This deposit was made with the permanent collection of the Northern Regional Research Laboratory, U.S. Department of Agriculture, Peoria, Illinois 61604, USA. Plasmid pSM1-17 confers ampicillin resistance to E. coli and contains a 6.8 Kbp HindIII DNA fragment that includes the δ-endotoxin gene from the 50 md plasmid of Bacillus thuringiensis HD73. Sufficient toxin is expressed from this gene in E. coli to make the intact cells toxic to cabbage loopers. A further modification of the DNA fragment was done to enhance toxin expression and to accomplish expression of toxin in an alternative host. Pseudomonas fluorescens. In order to eliminate unwanted DNA from the 6.8 Kbp fragment, pSM1-17 was digested with BamHI to open the circular plasmid at a site nearest to the 5' end of the toxin gene, and was treated with the exonuclease Bal31 to reduce the 6.8 Kbp HindII insert to about 2.9 Kbp. The free ends of the DNA were filled in with Klenow polymerase, BamHI linkers were added, and the linear DNA was ligated to reform the circular plasmid. The resultant vector, pFG270, was digested with XhoI to open the circular plasmid at a site nearest to the 3' end of the toxin gene. SalI linkers were added, the plasmid was ligated to its circular form, and the resultant vector, pFG10.6 was amplified in E. coli. pFG10.6 was digested completely with SalI and BamHI and the resulting 2.1 Kbp fragment containing the toxin gene was purified by gel electrophoresis and ligated into the BamHI and SalI sites of plasmid pRO1614. The resultant plasmid, pCH2.1, was amplified in E. coli and used to transform Pseudomonas fluorescens to carbenicillin resistance and the ability to synthesize δ-endotoxin. Pseudomonas fluorescens (pCH2.1) was deposited with NRRL and was given the accession number NRRL B-15977. The deposits NRRL B-15976 and NRRL B-15977 were made on 2nd July 1985. In the following illustrative experiments P. fluorescens (pCH2.1) was used in conjunction with controls of untransformed cells.

Example 5--Treatment and Testing of Microbes Hosting a Heterologous Gene.

The Pseudomonads were either killed by 1% lugol's iodine (100g KI, 50 g $I_2$, 100 ml glacial acetic acid in 1 L sterile distilled water) or by treatment with a 2% formalin solution, each at ambient temperatures.

Cell pellets from two liter broth cultures of P. fluorescens with and without B.t. toxin are divided in half. Half the cells are treated with 1% lugol's iodine for 4 hr, while the other half are held on ice. Washed lugol's treated cells and untreated cells are repelleted in 10 ml of sterile deionized water. Nine ml of this suspension ($10^8$-$10^{12}$ cells/ml) are sprayed on three young lettuce plants. The three sprayed plants are placed in a single enclosed chamber and 50-75 Trichoplusia ni larvae are applied to the plants. Plants are considered protected if there is no visible loss of foliage over the observation period.

TABLE 3

| Plant Assay 1 - P. fluorescens | | | | |
|---|---|---|---|---|
| Microorganism | Treatment | # Viable Cells/ml | Total # Larvae | Protection |
| P. fluorescens | Live | $2.5 \times 10^{11}$ | 75 | Not protected |
| P. fluorescens + Bt Toxin | Live | $3.1 \times 10^{11}$ | 75 | Protected |
| P. fluorescens | 1% Lugol's Iodine 4 hrs | 0 | 75 | Not protected |
| P. fluorescens + Bt Toxin | 1% Lugol's Iodine 4 hrs | 0 | 75 | Protected |

* Set up day 1, 25 2-day larvae applied to plants,
day 3, 50 5-day larvae applied to plants,
day 10, observation reported.

In the next study, newly hatched cabbage loopers are placed in a Petri dish containing droplets of the material to be bioassayed. The larvae imbibe the liquid and are then removed to small cups containing larval diet. The larvae are examined after seven days and the total number of animals killed is recorded. The following Table 4 indicates the results.

10

TABLE 4:  Bioassay 1 - P. fluorescens

| Microorganism | Treatment | # Larvae Killed/ Total | % Larvae Killed | Viable Cell Count/ml |
|---|---|---|---|---|
| P. fluorescens | Live | 0/15 | 0 | $8 \times 10^{11}$ |
| P. fluorescens + Bt Toxin | Live | 11/18 | 62 | $2.5 \times 10^{12}$ |
| P. fluorescens | 1% Lugol's 4 hrs | 0/15 | 0 | 0 |
| P. fluorescens + Bt Toxin | 1% Lugol's 4 hrs | 8/13 | 62 | 0 |

Bioassay 2 - P. fluorescens

| Microorganism | Treatment | # Larvae Killed/ Total | % Larvae Killed | Viable Cell Count/ml |
|---|---|---|---|---|
| P. fluorescens | Live | 0/15 | 0 | $3.7 \times 10^{11}$ |
| P. fluorescens + Bt Toxin | Live | 3/20 | 15 | $4.5 \times 10^{11}$ |
| P. fluorescens | 1% Lugol's 4 hrs | 0/15 | 0 | 0 |
| P. fluorescens + Bt Toxin | 1% Lugol's 4 hrs | 8/15 | 53 | 0 |

11

### Bioassay 3 - P. fluorescens

| Microorganism | Treatment | # Larvae Killed/ Total | % Larvae Killed | Viable Cell Count/ml |
|---|---|---|---|---|
| P. fluorescens + Bt Toxin | Live-frozen 14 days | 9/10 | 90 | $2.5 \times 10^{12}$ |
| P. fluorescens | 1% Lugol's 4 hrs | 1/15 | 7 | 0 |
| P. fluorescens + Bt Toxin | 1% Lugol's | 11/15 | 73 | 0 |
| P. fluorescens | 2% formalin 4 hrs | 0/15 | 0 | 0 |
| P. fluorescens + Bt Toxin | 2% formalin 4 hrs | 10/15 | 67 | 0 |

### Bioassay 4 - P. fluorescens

| Microorganism | Treatment | # Larvae Killed/ Total | % Larvae Killed | Viable Cell Count/ml |
|---|---|---|---|---|
| P. fluorescens | Live | 6/14 | 30 | $3 \times 10^{11}$ |
| P. fluorescens + Bt Toxin | Live | 20/20 | 100 | $3 \times 10^{10}$ |
| P. fluorescens | 1% Lugol's 4 hrs | 0/20 | 0 | 0 |
| P. fluorescens + Bt Toxin | 1% Lugol's 4 hrs | 18/20 | 90 | 0 |

In the next study, different methods of killing the cells were employed to determine the effect on cell stability to sonication. P. fluorescens strain 33 is an untransformed cell not containing the Bt toxin, while strain pCH contains the 2.1 kb toxin gene. In the first study, stationary phase cultures of strain 33 and strain pCH were harvested by centrifugation and cell pellets suspended in sterile deionized water at concentrations of $10^{10}$ and $10^9$, respectively. Aliquots of cells were exposed to a 70°C water bath for varying amounts of time and cell viability measured by plate counts on King's B agar. (See King, E.O. et al.(1954) J. Lab. & Clin. Med. 44:304). The medium has the following ingredients: Proteose peptone No. 3, 2.0 percent; Bacto agar, 1.5 percent; glycerol, C.P., 1.0 percent; $K_2HPO_4$ [anhydrous], 0.15 percent; $MgSO_4 \cdot 7H_2O$, 0.15 per cent; adjusted to pH 7.2.) Strain 33 was substantially completely killed within 5 min, while strain pCH showed substantially no viable cells within 10 min.

Cells of strain 33 (untreated, heat treated at 70°C, or lugol's treated [1% lugol's iodine, 2 hr, room temperature]) were suspended in 10 ml deionized water to give a cell concentration of approximately $10^9$/ml.

The cell suspension was then subjected to sonication for 5 min on a Bronson 200 Sonifier (using a microprobe output 10, 50% duty cycle, pulse mode). The optical density (575 nm) of the cell suspension was measured before and after the sonication treatment. A decrease in optical density indicates cell disruption. The following Tables 5 and 6 give the results.

TABLE 5:  Sonication – P. fluorescens 33

| Treatment | Optical Density (575nm) | |
| --- | --- | --- |
| | Pre-sonication | Post-sonication |
| Live | 0.24 | 0.05 |
| Lugol's (1%, 2hr, room temp) | 0.20 | 0.19 |
| Heat 70°C | | |
| 1 | 0.22 | 0.06 |
| 3 | 0.21 | 0.07 |
| 5 | 0.22 | 0.09 |
| 15 | 0.20 | 0.10 |
| 30 | 0.21 | 0.11 |
| 60 | 0.19 | 0.13 |
| 90 | 0.22 | 0.13 |

Cells of strain pCH ($5\times10^{10}$/ml) were heat treated in a 70°C water bath and assayed for toxicity against T. ni larvae. The bioassay procedure involved newly hatched cabbage loopers which were placed in a petri dish containing droplets of the materials to be bioassayed. The larvae were allowed to imbibe the liquid and were then removed to small cups containing larval diet. The larvae were examined after six days and the total number of animals killed recorded.

TABLE 6

| Bioassay Heat Treated P. fluorescens | | | |
| --- | --- | --- | --- |
| Treatment (70°C) Time (min) | # Viable Cells/Total Cells | # Larvae Killed/ Total Larvae | |
| | | lx | % Killed |
| 3 | $2 \times 10^5$ / $5.5 \times 10^{10}$ | 8/8 | 100 |
| 5 | $2 \times 10^6$ / $5.5 \times 10^{10}$ | 8/8 | 100 |
| 10 | $0$ / $5.5 \times 10^{10}$ | 8/8 | 100 |

Analysis of the persistence of the lugol's treated cells of P. fluorescens + B.t. toxin (strain pCH) was done in the greenhouse by (1) spraying leaves of Romaine lettuce with the pesticidal cells, (2) exposing the treated plants to the greenhouse environment for up to 19 days (the greenhouse was covered with a UV transparent roof), and (3) assaying the cabbage looper pesticidal activity remaining on the leaves with the

13

leaf inhibition assay. Measurement of B.t. toxin by feeding inhibition is as described previously, and the method for spraying the plants is described in Example 5. In this experiment a toxin activity level of 1.0 is equivalent to the pesticidal activity achieved with 0.19 g of Dipel/100 ml of spray applied at a rate of approximately 10 ml/plant (i.e., just prior to run-off).

As shown in Table 7, after 11 days the Pseudomonas preparation is still fully active whereas the Dipel example has lost over 2/3 of its initial activity. We interpret these data to mean that the fixed Pseudomonas cell is in some manner protecting the pesticidal toxin from inactivation on the leaf surface.

Table 7

| Greenhouse Persistence data | | |
|---|---|---|
| Activity | | |
| Day | pCH | Dipel |
| 1 | 0.75 | 1.0 |
| 5 | 1.15 | 0.85 |
| 8 | 1.1 | 1.2 |
| 11 | 1.0 | 0.3 |
| 19 | 0.13 | <0.01 |

It is evident from the above results that the killed bacteria containing toxin provide many advantages, while still retaining all or a substantial proportion of the pesticidal activity of the live bacteria. Maintenance of dead bacteria for storage, transportation and application is more convenient and economical than employing the live organisms. At the same time, the organisms provide encapsulated protection of the toxins, so as to maintain the toxins' bioactivity for long periods of time. In addition, denaturation of the proteases prevents proteolytic degradation of the polypeptide pesticides. Various means for killing the organisms without disrupting their structure or denaturing the protein pesticide can be employed to provide the desired pesticidal activity, while providing for a convenient pesticidal composition for use in formulation and application to plants and plant products.

**Claims**

1. intact, non-reproducing microbial cells containing a pesticide which is a polypeptide, is intracellular and is produced naturally or as a result of a heterologous gene in the cell, characterised in that the cells are stabilised such that the pesticidal activity is prolonged when the cell is applied to the environment of the target pest.

2. Cells according to claim 1, which are either prokaryotes selected from Enterobacteriaceae, Bacillaceae, Rhizobiaceae, Spirillaceae, Lactobacillaceae, Pseudomonadaceae, Azotobacteraceae and Nitrobacteraceae, or lower eukaryotes selected from Phycomycetes, Ascomycetes and Basidiomycetes.

3. Cells according to claim 2, which are a prokaryotic pesticide-producing strain of Bacillus thuringiensis, in which the strain is (B. thuringiensis) M-7, var. kurstaki HD1, HD2, var. finitimus HD3, var. alesti HD4, var. kurstaki HD73, var. sotto HD770, var. dendrolimus HD7, var. kenyae HD5, var. galleriae HD29, var. canadensis HD224, var. entomocidus HD9, var. subtoxicus HD109, var. aizawai HD11, var. morrisoni HD12, var. ostriniae HD501, var. tolworthi HD537, var. darmstadiensis HD146, var. toumanoffi HD201, var. kyushuensis HD541, var. thompsoni HD542, var. pakistani HD395, var. israelensis HD567, var. indiana HD521, var. dakota, var. tohokuensis HD866, var. kumanotoensis HD867, var. tochigiensis HD868, var. colmeri HD847, var. wuhanensis HD525 or var. tenebrionsis.

4. Cells according to claim 2, which are a prokaryotic species of Bacillus selected from B. cereus, B. moritai, B. popilliae, B. lentimorbus and B. sphaericus.

5. Cells according to claim 1, of a unicellular microorganism.

6. Cells according to claim 5, wherein the microorganism is a Pseudomonad containing a toxin derived from a strain of B. thuringiensis.

14

7. Cells according to any preceding claim, which have been killed under protease-deactivating or cell wall-strengthening conditions, while retaining pesticidal activity.

8. A method for protecting plants, which comprises applying to the plants cells according to any preceding claim.

**Revendications**

1. Cellules microbiennes intactes, non reproductrices, contenant un pesticide qui est un polypeptide, est intracellulaire et est produit naturellement ou à la suite de la présence d'un gène hétérologue dans la cellule, caractérisées en ce que les cellules sont stabilisées de telle sorte que l'activité pesticide est prolongée lorsque la cellule est appliquée à l'environnement du parasite cible.

2. Cellules selon la revendication 1, qui sont soit des procaryotes sélectionnés parmi les Enterobacteriaceae, Bacillaceae, Rhizobiaceae, Spirillaceae, Lactobacillaceae, Pseudomonadaceae, Azotobacteraceae et Nitrobacteraceae, ou des eucaryotes inférieurs sélectionnés parmi les Phycomycètes, les Ascomycètes et les Basidiomycètes.

3. Cellules selon la revendication 2, qui sont une souche procaryote, productrice de pesticide de Bacillus thuringiensis, dans lesquelles la souche est (B. thuringiensis) M-7, var. kurstaki HD1, HD2, var. finitimus HD3, var. alesti HD4, var. kurstaki HD73, var. sotto HD770, var. dendrolimus HD7, var. kenyae HD5, var. galleriae HD29, var. canadensis HD224, var. entomocidus HD9, var. subtoxicus, HD109, var. aizawai HD11, var. morrisoni HD12, var. ostriniae HD501, var. tolworthi HD537, var. darmstadiensis HD146, var. toumanoffi HD201, var. kyushuensis HD541, var. thompsoni HD542, var. pakistani HD395, var. israelensis HD567, var. indiana HD521, var. dakota, var. tohokuensis HD866, var. kumanotoensis HD867, var. tochigiensis HD868, var. colmeri HD847, var. wuhanensis HD525 ou var. tenebrionsis.

4. Cellules selon la revendication 2, qui sont une espèce procaryote de Bacillus sélectionnée parmi B. cereus, B. moritai, B. popilliae, B. lentimorbus et B. sphaericus.

5. Cellules selon la revendication 1, d'un micro-organisme unicellulaire.

6. Cellules selon la revendication 5, dans lesquelles le micro-organisme est un Pseudomonas contenant une toxine provenant d'une souche de B. thuringiensis.

7. Cellules selon l'une des revendications précédentes, qui ont été tuées dans des conditions de désactivation des protéases ou de renforcement de la paroi cellulaire, tout en conservant leur activité pesticide.

8. Procédé de protection de végétaux, comprenant l'application aux végétaux de cellules selon l'une des revendications précédentes.

**Patentansprüche**

1. Intakte, nicht-reproduzierende mikrobielle Zellen, die ein Pestizid enthalten, welches ein Polypeptid ist, intrazellulär vorliegt und natürlich oder als Ergebnis eines heterologen Gens in der Zelle erzeugt wird, dadurch gekennzeichnet, daß die Zellen so stabilisiert sind, daß die Pestizid-Aktivität verlängert ist, wenn die Zelle in der Umgebung des Zielschädlings ausgebracht wird.

2. Zellen nach Anspruch 1, die entweder aus Enterobacteriaceae, Bacillaceae, Rhizobiaceae, Spirillaceae, Lactobacillaceae, Pseudomonadaceae, Azotobacteraceae und Nitrobacteraceae ausgewählte Prokaryoten sind, oder niedere Eukaryoten, die aus Phycomycetes, Ascomycetes und Basidiomycetes ausgewählt sind.

3. Zellen nach Anspruch 2, die einen prokaryotischen Pestizid-erzeugenden Stamm von Bacillus thuringiensis repräsentieren, wobei der Stamm (B. thuringiensis) M-7, var. kurstaki HD1, HD2, var. finitimus HD3, var. alesti HD4, var. kurstaki HD73, var. sotto HD770, var. dendrolimus HD7, var. kenyae HD5, var. galleriae HD29, var. canadensis HD224, var. entomocidus HD9, var. subtoxicus HD109, var. aizawai HD11, var. morrisoni HD12, var. ostriniae HD501, var. tolworthi HD537, var. darmstadiensis HD146, var. toumanoffi HD201, var. kyushuensis HD541, var. thompsoni HD542, var. pakistani HD395, var. israelensis HD567, var. indiana HD521, var. dakota, var. tohokuensis HD866, var. kumanotoensis HD867, var. tochigiensis HD868, var. colmeri HD847, var. wuhanensis HD525 oder var. tenebrionsis ist.

**4.** Zellen nach Anspruch 2, die eine prokaryotische Bacillus -Spezies repräsentieren, die aus B. cereus, B. moritai, B. popilliae, B. lentimorbus und B. sphaericus ausgewählt ist.

**5.** Zellen nach Anspruch 1 eines einzelligen Mikroorganismus.

**6.** Zellen nach Anspruch 5, worin der Mikroorganismus Pseudomonas ist, der ein von einem B. thuringiensis-Stamm abgeleitetes Toxin enthält.

**7.** Zellen nach irgendeinem vorhergehenden Anspruch, welche unter Protease-deaktivierenden oder Zellwand-verstärkenden Bedingungen abgetötet worden sind, wobei die Pestizid-Aktivität erhalten bleibt.

**8.** Verfahren zum Schutz von Pflanzen, umfassend das Aufbringen von Zellen nach irgendeinem der vorhergehenden Ansprüche auf die Pflanzen.